# EUROPÄISCHE PATENTSCHRIFT

(11) **EP 2 934 490 B1**
(45) Veröffentlichungstag und Bekanntmachung des Hinweises auf die Patenterteilung: **12.08.2020**
(21) Anmeldenummer: 13805906.8
(22) Anmeldetag: 17.12.2013
(51) Int. Cl.: A61K 9/20, A61K 45/06, A61K 31/4985, A61K 31/216, A61K 31/27, A61K 31/506

(54) **TABLETTEN MIT VERBESSERTER AKZEPTANZ UND GUTER LAGERSTABILITÄT**
TABLETS WITH IMPROVED ACCEPTANCE AND GOOD STORAGE STABILITY
COMPRIMÉS PRÉSENTANT UNE APPÉTENCE AMÉLIORÉE ET UNE STABILITÉ AU STOCKAGE SATISFAISANTE

(30) Priorität: 19.12.2012 EP 12198101
(43) Veröffentlichungstag der Anmeldung: 28.10.2015
(73) Patentinhaber: Bayer Animal Health GmbH, 51368 Leverkusen (DE)
(72) Erfinder: KANIKANTI, Venkata-Rangarao, 51381 Leverkusen (DE); HAMANN, Hans-Jürgen, 41539 Dormagen (DE); SCHULTE, Georg, 42109 Wuppertal (DE); BILLIAN, Patrick, 51371 Leverkusen (DE)
(74) Vertreter: Tier 1 Patents
(86) Internationale Anmeldenummer: PCT/EP2013/076878
(87) Internationale Veröffentlichungsnummer: WO 2014/095845

(56) Entgegenhaltungen:
- EP-A1- 2 080 515
- WO-A1-2012/049156
- DE-A1-102008 022 520
- US-A1- 2005 203 097
- US-A1- 2012 046 296
- "VETERINARY COMPOSITIONS", IP.COM JOURNAL, IP.COM INC., WEST HENRIETTA, NY, US, 22 July 2008 (2008-07-22), XP013125858, ISSN: 1533-0001

## Beschreibung

Die vorliegende Erfindung betrifft Tabletten für Tiere, mit verbesserter Akzeptanz und guter Lagerstabilität.

Die Verabreichung von Tabletten an Tiere ist problematisch, da diese für die Tiere ohne jede Attraktivität sind und von ihnen in der Regel nur unfreiwillig aufgenommen werden. Üblicherweise müssen die Tabletten in Futter verpackt werden, um sie zu applizieren. Hierbei ist nicht immer garantiert, dass die Arznei vollständig und damit in der richtigen Dosierung appliziert werden kann. Auch das Freisetzungsprofil des Arzneimittels kann sich bei der Gabe im Futter ändern.

Aus EP279343 sind bereits Wirkstoffkombinationen aus Phenylguanidinen oder Benzimidazolen und Tetrahydropyrimidinen sowie deren Formulierungen bekannt.

WO 2005/000275 beschreibt Tabletten die Enrofloxacin und Geschmacks- oder Aromastoffe enthalten.

Aus WO 2012/049156 sind stärkefreie kaubare Formulierungen ("chewables") bekannt, die Aromastoffe enthalten.

In WO 95/20942 werden für Tiere - vor allem für Hunde - attraktive Köder beschrieben, in die Arzneimittel eingebracht und so verabreicht werden können.

Kaubare Tabletten, die zur Verabreichung von Anxiolytika an Haustiere dienen, sind in WO 2010/132286 beschrieben.

Wie sich z. B. aus einigen oben genannten Dokumenten ergibt, ist es im Prinzip bereits bekannt, dass die Palatabilität durch Zugabe geeigneter Aromen und/oder Geschmacksstoffe gesteigert werden kann. Allerdings werden durch diesen Zusatz die Eigenschaften der Tabletten häufig in einem in der Praxis nicht akzeptablen Maß verschlechtert. Beispielsweise haben Tabletten mit einem hohen Anteil an Fleischaroma die Neigung, ihre Eigenschaften während der Lagerung zu verändern. Dies liegt insbesondere an der Alterung des Fleischaromas. Durch die Alterungsprozesse des Fleischaromas kann sich z.B. die Zerfallszeit der Tabletten in einem für Arzneimittel nicht akzeptablen Maß verändern. Bei Tabletten, die kein Fleischaroma enthalten, ist das Erreichen einer guten Lagerstabilität in der Regel nicht schwierig. Der Einsatz von Fleischaroma in solchen Tabletten bringt jedoch die oben angesprochenen Schwierigkeiten mit sich.

Es besteht daher Bedarf an gut palatablen Tabletten, welche die Anforderungen an Arzneimittel erfüllen und insbesondere eine gute Lagerstabilität aufweisen.

Die Erfindung betrifft eine Tablette, enthaltend:
- Febantel, Praziquantel und Pyrantel als pharmazeutische Wirkstoffe,
- mindestens 28 Gew.-% Fleischaroma,
- mindestens 2 Gew.-% Croscarmellose-Natrium als Stabilisierungsmittel und
- Stärke oder eine modifizierte Stärke, wobei die modifizierte Stärke ausgewählt ist aus der Gruppe umfassend physikalisch vorbehandelter Stärken, Hydroxyethylstärke, Hydroxypropylstärke, Methylstärke, Carboxymethylstärke, Stärkeacetat, Hydroxypropylstärkeacetat, Hydroxyethylstärkeacetat, Stärkephosphate, Stärkesulfate, Distärkephosphate, Phosphate hydroxypropylierter Stärken, Stärkedicarbonsäurediester und Salze anionischer Stärkederivate.

Die Wirkstoffe sind solche gegen Parasiten (Ektoparasiten und/oder Endoparasiten), wie anthelmintische Wirksto ffe. erfindungsmäßen Tabletten enthalten eine Dreierkombination aus Pyrantel-Embonat und Praziquantel.

Die Wirkstoffe können auch - soweit zutreffend - in Form ihrer Salze mit pharmazeutisch annehmbaren Säuren oder Basen oder auch als Solvate, insbesondere Hydrate, der Wirkstoffe oder ihrer Salze eingesetzt werden.

Auch Prodrugs der Wirkstoffe können verwendet werden.

Erfindungsgemäß enthalten die Tabletten die Wirkstoffe in einer pharmazeutisch wirksamen Menge, wobei "pharmazeutisch wirksame Menge" für eine nicht toxische Menge Wirkstoff steht, die den gewünschten Effekt bewirken kann. Die eingesetzte Wirkstoffmenge hängt vom Wirkstoff, dem behandelten Tier und von Art, Schwere und Stadium der Krankheit ab.

Im Allgemeinen enthalten die Tabletten etwa 0,0001 bis 50 Gew.-% an Wirkstoffen. Die Tabletten können 0,01 bis 40 Gew.-%, 0,1 bis 35 Gew.-%, 1 bis 30 Gew.-%, 5 bis 30 Gew.-% oder 10 bis 30 Gew.-% Wirksto enthalten. In weiteren Ausgestaltungen können die Tabletten auch 1 bis 35 5 bis 35 Gew.-% oder 10 bis 35 Gew.-% Wirksto ffe enthalten.

Die Wirkstoffmenge kann auch als Gewicht pro Tablette angegeben werden, z.B. mindestens 5 mg, mindestens 10 mg, mindestens 20 mg, mindestens 30 mg, mindestens 40 mg, mindestens 50 mg, oder mindestens 100 mg Wirkstoffe . Beispielsweise können die Tabletten 5 bis 2000 mg, 10 bis 1500 mg, 10 bis 1000 mg, 10 bis 500 mg, 20 bis 2000 mg, 20 bis 1500 mg, 20 bis 1000 mg, 20 bis 500 mg, 50 bis 2000 mg, 50 bis 1500 mg, 50 bis 1000 mg oder 50 bis 500 mg an Wirksto ffen enthalten. Febantel wird vorzugsweise in Konzentrationen von 9 bis 20 Gew.-%, bevorzugt 11 bis 17 Gew.-%, besonders bevorzugt 12 bis 16 Gew.-% eingesetzt.

Praziquantel wird vorzugsweise in Konzentrationen von 1 bis 10 Gew.-%, bevorzugt 2 bis 8 Gew.-%, besonders bevorzugt 3 bis 7 Gew.-% eingesetzt.

Pyrantel, insbesondere sein Embonat, wird vorzugsweise in Konzentrationen von 8 bis 20 Gew.-%, bevorzugt 9 bis 17-Gew.-%, besonders bevorzugt 11 bis 15 Gew.-% eingesetzt.

Die erfindungsgemäßen Tabletten enthalten Fleischaroma. Fleischaroma bezeichnet einen Zusatz, der synthetischen oder tierischen Ursprungs oder eine Mischung von beidem ist und den Tabletten einen fleischähnlichen Geruch und/oder Geschmack verleiht. Vorzugsweise werden Fleischaromen rein tierischen Ursprungs eingesetzt. Diese werden z.B. aus Rindfleisch, Geflügel, Fisch, Tierhäuten oder Tierlebern hergestellt. Bevorzugt sind sogenannte Trockenleberpulver, z.B. aus Rind, Schaf, Geflügel oder Schwein und besonders bevorzugt aus Geflügel oder Schwein.

Das Fleischaroma wird vorzugsweise in einer Menge von mindestens 28 Gew.-%, bevorzugt mindestens 30 Gew.-%, besonders bevorzugt mindestens 31 Gew.-% eingesetzt. Üblicherweise werden nicht mehr als 40 Gew.-% Fleischaroma eingesetzt, bevorzugt 30 bis 35 Gew.-% (Prozentangaben sind hier wie auch anderswo Gewichtsprozent der fertigen Tablette, soweit nicht anders angegeben).

Optional können zusätzlich Geschmacksverstärker wie z.B. Hefe, Hefeextrakte oder Glutamat in üblichen Mengen eingesetzt werden, z.B. in Konzentrationen von 1 bis 30 Gew.-%, bevorzugt 1 bis 20 Gew.-%.

Weiterhin enthalten die erfindungsgemäßen Tabletten ein Stabilisierungsmittel. Darunter soll hier ein Hilfsstoff verstanden werden, der die Lagerfähigkeit der Tabletten verbessert. Wie schon erläutert, ist das Stabilisierungsmittel insbesondere im Hinblick auf den hohen Anteil an Fleischaroma erforderlich. Es soll vor allem die im Zusammenhang mit den hohen Anteilen an Fleischaroma auftretenden Alterungsprozesse verhindern oder verringern. Als geeignet haben sich wasserlösliche Komponenten erwiesen, die eine gewisse Wirkung als Sprengmittel aufweisen. ist Croscarmellose-Natrium. Die erfindungsgemäßen Tabletten enthalten das Stabilisierungmittel in einem Anteil von mindestens 2 Gew.-%, üblicherweise 2 bis 15 Gew.-%, bevorzugt 2 bis 10 Gew.-%, besonders bevorzugt 3 bis 9 Gew.-%. Gemäß einer Ausfuhrungsform genügen schon relativ geringe Mengen wie 3 bis 8 Gew.-%.

Die erfindungsgemäßen Tabletten können weitere Hilfsstoffe enthalten:
Bevorzugt enthalten die erfindungsgemäßen Tabletten Stärke oder ein Stärkederivat als Füllmittel, das auch in gewisser Weise wie ein Sprengmittel wirkt. Stärke kann beispielsweise Stärke aus Weizen, Reis, Mais, Tapioka, Roggen, Hafer oder Kartoffeln sein. Modifizierte Stärken können physikalisch vorbehandelte Stärken wie vorgekochte Stärke oder chemisch veränderte Stärken wie Hydroxyethylstärke, Hydroxypropylstärke, Methylstärke, Carboxymethylstärke, Stärkeacetat, Hydroxypropylstärkeacetat, Hydroxyethylstärkeacetat, Stärkephosphate, Stärkesulfate, oder chemisch oder ionisch vernetzte Stärken wie Distärkephosphate, Phosphate hydroxypropylierter Stärken, Stärkedicarbonsäurediester oder Salze anionischer Stärkederivate sein. Bevorzugt ist Stärke, wie z. B. Maisstärke, als Füllmittel vorhanden, und zwar in Mengen von üblicherweise 5 bis 30 Gew.-%, vorzugsweise 8 bis 20 Gew.-%, besonders bevorzugt 10 bis 15 Gew.-% bezogen auf das Gesamtgewicht der Tablette.

Die erfindungsgemäßen Tabletten enthalten weiterhin einen weiteren Füllstoff, wie mikrokristalline Cellulose, Maltodextrin; einen Zucker wie Saccharose, Traubenzucker oder Lactose; anorganische Füllstoffe, wie Calciumcarbonat, Dicalciumphosphat oder Magnesiumcarbonat. Vorzugsweise wird mikrokristalline Cellulose oder insbesondere Lactose eingesetzt. Lactose ist ein handelsüblicher Arzneimittelhilfsstoff, der in verschiedenen Formen erhältlich ist, z.B. sprühgetrocknet oder als wasserfreie Lactose. Bevorzugt wird erfindungsgemäß Lactose-Monohydrat eingesetzt (z.B. Milchzucker fein der Fa. DMV International). Die erfindungsgemäßen Tabletten enthalten 5 bis 20 Gew.-% Lactose, bevorzugt 6 bis 15 Gew.-%, besonders bevorzugt 8 bis 12 Gew.-% bezogen auf das Gesamtgewicht der Tablette.

Die erfindungsgemäßen Tabletten enthalten vorzugsweise mikrokristalline Cellulose oder einen vergleichbaren Hilfsstoff. Mikrokristalline Cellulose ist ein handelsüblicher Arzneimittelhilfsstoff (z.B. Avicel® PH 101 der Fa. FMC). Die erfindungsgemäßen Tabletten enthalten 2 bis 10 Gew.-%, bevorzugt 5 bis 10 Gew.-%, besonders bevorzugt 5,5 bis 8 Gew.-% bezogen auf das Gesamtgewicht der Tablette. In einer alternativen Ausgestaltung enthalten die Tabletten bevorzugt 3 bis 8 Gew.-% und besonders bevorzugt 4 bis 6 Gew.-% bezogen auf das Gesamtgewicht der Tablette.

Bevorzugt können die erfindungsgemäßen Tabletten Siliciumdioxid enthalten, insbesondere kolloidales wasserfreies Siliciumdioxid, in Mengen von 0,01 bis 0,3 Gew.-%, insbesondere 0,05 bis 0,2 Gew.-% bezogen auf das Gesamtgewicht der Tablette.

Die Tabletten können weitere übliche pharmazeutische Hilfsstoffe enthalten. Als solche seien beispielhaft genannt: Schmier- und Gleitmittel wie z.B. Magnesiumstearat, Stearinsäure, Talkum, Bentonite; Bindemittel wie z.B. Stärke, Gelatine, Celluloseether oder lineares Polyvinylpyrrolidon sowie Trockenbindemittel wie mikrokristalline Cellulose.

Bevorzugt enthalten die erfindungsgemäßen Tabletten ein Schmiermittel, insbesondere Magnesiumstearat, in Mengen von 0,1 bis 1,0 Gew.-%, bevorzugt 0,1 bis 0,5 Gew.-% bezogen auf das Gesamtgewicht der Tablette.

Weiterhin können die erfindungsgemäßen Tabletten ein Bindemittel, wie z. B. Povidone enthalten. Povidone bezeichnet hydrophile Polyvinylpyrrolidon-Polymere, wobei als Bindemittel vorzugsweise solche mit einem K-Wert von 30 oder kleiner eingesetzt werden. Povidone wird in Konzentrationen von 0,5 bis 5 Gew.-%, bevorzugt 1 bis 3 Gew.-% eingesetzt.

Weiterhin können die erfindungsgemäßen Tabletten Natriumlaurylsulfat oder einen vergleichbaren Hilfsstoff enthalten. Natriumlaurylsulfat wird in Konzentrationen von 0,05 bis 1 Gew.-%, bevorzugt 0,1 bis 0,3 Gew.-% eingesetzt.

Falls dies für weitere therapeutische Aktivitäten benötigt wird, kann in die Tablette ein weiterer Wirkstoff hinzugefügt werden. Sollte dieser Wirkstoff mit anderen Inhaltsstoffen der Tablette nicht kompatibel sein, kann sein Granulat als eine separate Schicht auf- bzw. eingebracht werden. Auf diese Weise kann eine 2-schichtige Tablette hergestellt werden.

Weiterhin kann die erfindungsgemäße Tablette zumindest ein Antioxidans enthalten, um die Oxidation der Wirkstoffe zu vermeiden. Beispiele für Antioxidantien sind Butylhydroxyltoluene (BHT) und Propylgallat.

Die Tabletten lassen sich herstellen nach einem Verfahren, bei dem man
(a) die Wirkstoffe, sowie gegebenenfalls weitere Hilfsstoffe mischt, granuliert und das Granulat gegebenenfalls mahlt,
(b) der Mischung aus (a) das Fleischaroma sowie gegebenenfalls weitere Hilfsstoffe zusetzt und alles zu einer homogenen tablettierbaren Mischung verarbeitet und
(c) die Mischung anschließend zu Tabletten verarbeitet.

Die erfindungsgemäßen Tabletten lassen sich herstellen nach einem Verfahren, bei dem man
(a) die Wirkstoffe, sowie gegebenenfalls weitere Hilfsstoffe mischt, granuliert und das Granulat gegebenenfalls siebt,
(b) das Fleischaroma gegebenenfalls mit weiteren Hilfsstoffen homogen mischt und trockengranuliert,
(c) der Mischung aus (a) und (b) gegebenenfalls weitere Hilfsstoffe zusetzt und alles zu einer homogenen tablettierbaren Mischung verarbeitet und
(d) die Mischung anschließend zu Tabletten verarbeitet.

Herstellungsschritte (a) können als Nassgranulation durchgeführt werden. Alternativ können Herstellungsschritte (a) als Trockengranulation durchgeführt werden, dann werden die fehlenden Komponenten in einer separaten Nassgranulation verarbeitet. Es können auch alle Komponenten mit Croscarmellose-Natrium in einem Schritt trocken granuliert werden. Danach mischt man beispielsweise mit Magnesiumstearat und kolloidalem Siliziumdioxid, um eine tablettierbare Mischung zu bekommen.

Die erfindungsgemäßen Tabletten werden zur einfachen Verabreichung oral applizierbarer pharmazeutischer Wirkstoffe verwendet. Die erfindungsgemäßen Arzneimittel eignen sich daher zur Prophylaxe und Behandlung entsprechender Erkrankungen, wobei sie gemäß einer bevorzugten Ausführungsform bei der Bekämpfung von Endoparasiten, insbesondere Helminthen, bei Tieren angewendet werden. Die erfindungsgemäßen Mittel eignen sich generell zur Anwendung in der Tierhaltung und Tierzucht bei Nutz-, Zucht-, Zoo-, Labor-, Versuchs- und Hobbytieren. Vorzugsweise werden sie bei solchen Tieren eingesetzt, bei denen eine Verbesserung der Palatabilität durch den Fleischaromazusatz zu erwarten ist. Dies sind üblicherweise Fleischfresser.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere, wie z.B. Nerze, Chinchilla, Waschbär.

Zu Labor- und Versuchstieren gehören z.B. Mäuse, Ratten, Meerschweinchen, Goldhamster, Hunde und Katzen.

Zu den Hobbytieren gehören z.B. Hunde und Katzen.

Die erfindungsgemäßen Mittel werden besonders bevorzugt bei Hunden und Katzen, insbesondere Hunden eingesetzt.

Die erfindungsgemäßen Tabletten zeichnen sich durch eine ausgezeichnete Palatabilität aus. Die mechanischen Eigenschaften der Tabletten sind gut. Durch den erfindungsgemäßen Einsatz eines Stabilisierungsmittels gelang es auch, die Tabletten ausreichend lagerstabil zu machen. Tabletten, die kein Stabilisierungsmittel oder einen zu geringen Anteil davon enthalten, zeigen bei Lagerung eine Veränderung in der Zerfallskinetik: Die Zerfallszeiten dieser nicht erfindungsgemäßen Tabletten steigen deutlich an, während mit den erfindungsgemäßen Tabletten weitgehend konstante Zerfallszeiten über einen Lagerzeitraum von mindestens einem Jahr, in der Regel sogar zwei Jahren, bevorzugt über 3 Jahren erreicht werden können. Dies wird durch den erfindungsgemäßen Zusatz von Stabilisierungsmitteln erreicht, die in Anwesenheit der hohen Anteile an Fleischaroma den auftretenden Alterungsprozess verhindern können. Zerfallszeiten werden im Zweifel gemäß *"disintegration method 2.9.1 (Test B)"* der *European Pharmacopoeia* 6 bestimmt, wo auch zulässige Toleranzen für die Zerfallszeiten angegeben sind.

### Beispiele

| **Inhaltsstoffe** | **(1) mg** | **(2) mg** | **(3) mg** | **(4) mg** | **(5) mg** | **(6) mg** |
|---|---|---|---|---|---|---|
| Febantel | 150,0 | 525.0 | 150,0 | 525.0 | 450,0 | 375,0 |
| Praziquantel | 50,0 | 175,0 | 50,0 | 175,0 | 150,0 | 125,0 |
| Pyrantel embonate | 144,0 | 504,0 | 144,0 | 504,0 | 432,0 | 360,0 |
| Lactose-Monohydrat | 100,0 | 350,0 | 100,0 | 350,0 | 300 | 250,0 |
| Maisstärke | 143,0 | 500,5 | 143,0 | 500,5 | 429,0 | 357,5 |
| Povidone 25 | 18,00 | 63,0 | 18,00 | 63,0 | 54,0 | 45,0 |
| Natriumlaurylsulfat | 2,0 | 7,0 | 2,0 | 7,0 | 6,0 | 5,0 |
| Sprühgetrocknetes Lebermehl | 355,4 | 1243,9 | 355,4 | 1243,9 | 1066,2 | 888,5 |
| Mikrokristalline Cellulose | 49,0 | 171,5 | 49,0 | 171,5 | 147,0 | 122,5 |
| Natrium-Croscarmellose | 40,0 | 280,0 | 50,0 | 250,0 | 240,0 | 200,0 |
| Magnesiumstearat | 3,0 | 10,5 | 3,0 | 10,5 | 9,0 | 7,5 |
| Wasserfreies kolloidales Siliziumdioxid | 1,0 | 3,5 | 1,0 | 3,5 | 3,0 | 2,5 |
| 1 Tablettengewicht | 1055,4 | 3833.9 | 1065,4 | 3803,9 | 3286,2 | 2738,5 |

### Herstellverfahren:

### 1. Herstellungsschritt ("preblend")

In einem Mischgranulator werden Praziquantel, Febantel und Pyrantel-Embonat sowie ein Teil der Maisstärke und Lactose-Monohydrat gemischt. Die Mischung wird mit einer wässrigen Lösung von Povidone und Natriumlaurylsulfat granuliert, anschließend getrocknet und vorsichtig gesiebt.

### 2. Herstellungsschritt ("postblend")

Fleischaroma, restliche Maisstärke und die mikrokristalline Cellulose werden trocken gemischt, kompaktiert und gesiebt.

Bevorzugt weist das Fleischaroma hierbei einen Feuchtigkeitsgehalt von mindestens 5,5 % (Bestimmung gemäß Karl-Fischer-Titration) auf. Hierdurch wird ein Granulat mit einem akzeptablen Fließverhalten erhalten. Das liegt daran, dass Fleischaroma hygroskopisch ist und dadurch nicht gut fließt. Das Verwenden einer feuchten Granulierung ist weniger bevorzugt, da das Aroma dabei seine flüchtigen Komponenten während des Trocknungsschrittes verliert.

### 3. Herstellungsschritt ("final blend" und Tablettierung)

Die erforderlichen Mengen *preblend* und *postblend* werden mit Croscarmellose-Natrium, Magnesiumstearat und wasserfreiem Siliziumdioxid gemischt. Diese *final blend* wird dann zu Tabletten verarbeitet.

Alternativ kann Herstellungsschritt 1 als Trockengranulation durchgeführt werden.

Anstelle von Herstellungsschritt 1 und 2 können die Komponenten dieser beiden Schritte auch mit der Croscarmellose-Natrium in einem Schritt trocken granuliert werden und mit Magnesiumstearat und wasserfreiem Siliziumdioxid gemischt werden.

### Langzeittest der Zerfallseigenschaften

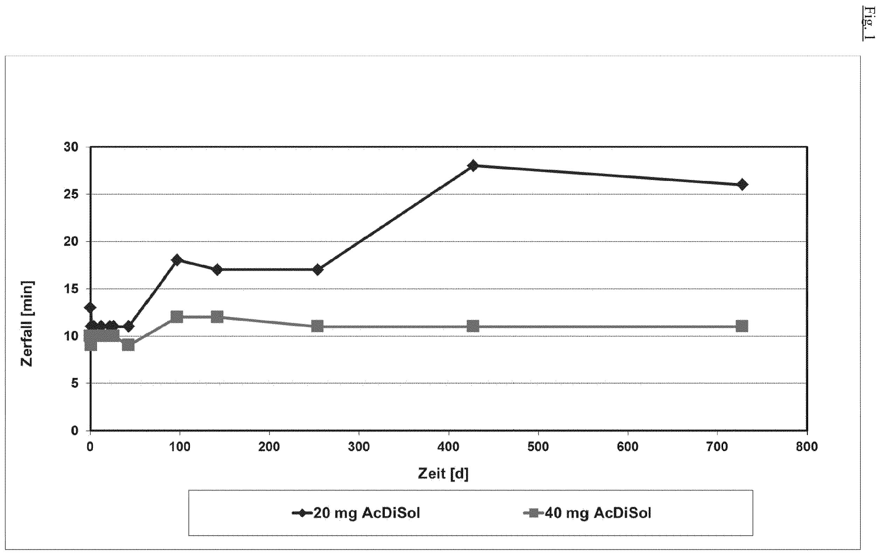

Fig. 1 zeigt die Veränderung der Zerfallszeiten während eines Stresstests bei 40°C von Formulierungen mit verschiedenen Croscarmellose-Natrium (AcDiSol) Mengen. Für die mit "40 mg AcDiSol" (40 mg Croscarmellose-Natrium) bezeichneten Daten wurden knochenförmige Tabletten der Zusammensetzung gemäß Beispiel 1 hergestellt. Im Vergleichsbeispiel wurde bis auf den geringeren Anteil von 20 mg Croscarmellose-Natrium ("20 mg AcDiSol") die Zusammensetzung beibehalten. Die Tabletten wurden bei 40°C über den in Tagen angegebenen Zeitraum ("Zeit [d]") gelagert, von Zeit zu Zeit wurden die Zerfallseigenschaften überprüft. Die Ergebnisse sind in Fig. 1 dargestellt.

Man erkennt hierbei, dass für die Tabletten mit 20 mg Croscarmellose-Natrium (AcDiSol) nach etwa 100 Tagen eine deutlich erhöhte Zerfallszeit vorliegt, die innerhalb eines Zeitraums von gut einem Jahr noch stärker ansteigt. Demgegenüber bleibt die Zerfallszeit der Tabletten mit 40 mg Croscarmellose-Natrium ("40 mg AcDiSol") über den gesamten Untersuchungszeitraum im Wesentlichen konstant. Da eine längere Zerfallszeit im vorliegenden Fall u.a. mit einer veränderten, hier verzögerten, Abgabe des Wirkstoffs und folglich einem insgesamt veränderten Wirkprofil gegenüber der ursprünglich hergestellten Tablette einhergeht, ist eine solche Erhöhung bzw. Abweichung von deutlichem Nachteil. Dieser Nachteil wird durch die entsprechend verbesserte Lagerstabilität durch den erhöhten Gehalt an Stabilisierungsmittel, hier beispielsweise mit einem Gehalt von 40 mg Croscarmellose-Natrium, beseitigt. Mit Bezug auf Beispiel 1 entspricht die Menge von 20 mg Croscarmellose-Natrium (AcDiSol) einem Gehalt von weniger als 2 Gew.-% bezogen auf das Gesamtgewicht der Tablette.

## Patentansprüche

1. Tablette enthaltend:
- Febantel, Praziquantel und Pyrantel als pharmazeutische Wirkstoffe,
- mindestens 28 Gew.-% Fleischaroma,
- mindestens 2 Gew.-% Croscarmellose-Natrium als Stabilisierungsmittel und
- Stärke oder eine modifizierte Stärke, wobei die modifizierte Stärke ausgewählt ist aus der Gruppe umfassend physikalisch vorbehandelter Stärken, Hydroxyethylstärke, Hydroxypropylstärke, Methylstärke, Carboxymethylstärke, Stärkeacetat, Hydroxypropylstärkeacetat, Hydroxyethylstärkeacetat, Stärkephosphate, Stärkesulfate, Distärkephosphate, Phosphate hydroxypropylierter Stärken, Stärkedicarbonsäurediester und Salze anionischer Stärkederivate.

2. Tablette gemäß Anspruch 1, enthaltend mindestens 30 Gew.-% Fleischaroma.

3. Tablette gemäß Anspruch 1 oder 2, enthaltend 3 bis 8 Gew.-% Croscarmellose-Natrium als Stabilisierungsmittel.

4. Tablette gemäß einem der vorstehenden Ansprüche, enthaltend Lactose.

5. Tablette gemäß einem der Ansprüche 1 bis 4, wobei Pyrantel als Pyrantelembonat vorliegt.

6. Tablette gemäß Anspruch 1 zur Verwendung zur Bekämpfung von Krankheiten bei Tieren.

7. Tablette gemäß einem der Ansprüche 1 bis 6 zur Verwendung zur Bekämpfung von Helminthen bei Tieren.

8. Verfahren zur Herstellung der Tabletten gemäß Anspruch 1, bei dem man
(a) die Wirkstoffe, sowie gegebenenfalls weitere Hilfsstoffe mischt, granuliert und das Granulat gegebenenfalls siebt,
(b) das Fleischaroma gegebenenfalls mit weiteren Hilfsstoffen homogen mischt und trockengranuliert,
(c) der Mischung aus (a) und (b) gegebenenfalls weitere Hilfsstoffe zusetzt und alles zu einer homogenen tablettierbaren Mischung verarbeitet und
(d) die Mischung anschließend zu Tabletten verarbeitet.

## Claims

1. Tablet containing:
- febantel, praziquantel and pyrantel as active pharmaceutical ingredients,
- at least 28% by weight of meat flavouring,
- at least 2% by weight of croscarmellose sodium as stabilizing agent and
- starch or a modified starch, the modified starch being selected from the group comprising physically pretreated starches, hydroxyethyl starch, hydroxypropyl starch, methyl starch, carboxymethyl starch, starch acetate, hydroxypropyl starch acetate, hydroxyethyl starch acetate, starch phosphates, starch sulfates, distarch phosphates, phosphates of hydroxypropylated starches, starch dicarboxylic diesters and salts of anionic starch derivatives.

2. Tablet according to Claim 1, containing at least 30% by weight of meat flavouring.

3. Tablet according to Claim 1 or 2, containing from 3 to 8% by weight of croscarmellose sodium as stabilizing agent.

4. Tablet according to any of the preceding claims, containing lactose.

5. Tablet according to any of Claims 1 to 4, wherein pyrantel is in the form of pyrantel embonate.

6. Tablet according to Claim 1 for use in controlling diseases in animals.

7. Tablet according to any of Claims 1 to 6 for use in controlling helminths in animals.

8. Method for preparing the tablets according to Claim 1, in which
(a) the active ingredients, and where required any further excipients, are mixed, granulated, and the granules screened if necessary,
(b) the meat flavouring is homogenously mixed and dry granulated possibly with further excipients,
(c) any further excipients are added to the mixture from (a) and (b) where required and everything is processed to form a homogeneous compressible mixture, and
(d) the mixture is subsequently processed to form tablets.

## Revendications

1. Comprimé contenant :
- du Fébantel, du Praziquantel et du Pyrantel en tant que principes actifs pharmaceutiques,
- au moins 28 % en poids d'arôme de viande,
- au moins 2 % en poids de croscarmellose sodique en tant qu'agent stabilisant et
- de l'amidon ou un amidon modifié, l'amidon modifié étant choisi dans le groupe comprenant les amidons physiquement prétraités, l'amidon hydroxyéthylé, l'amidon hydroxypropylé, l'amidon méthylé, l'amidon carboxyméthylé, l'acétate d'amidon, l'acétate d'amidon hydroxypropylé, l'acétate d'amidon hydroxyéthylé, les phosphates d'amidon, les sulfates d'amidon, les phosphates de diamidon, les phosphates d'amidons hydroxypropylés, les diesters d'acides dicarboxyliques d'amidon et les sels de dérivés anioniques d'amidon.

2. Comprimé selon la revendication 1, contenant au moins 30 % en poids d'arôme de viande.

3. Comprimé selon la revendication 1 ou 2, contenant 3 à 8 % en poids de croscarmellose sodique en tant qu'agent stabilisant.

4. Comprimé selon l'une quelconque des revendications précédentes, contenant du lactose.

5. Comprimé selon l'une quelconque des revendications 1 à 4, le pyrantel étant présent en tant qu'embonate de pyrantel.

6. Comprimé selon la revendication 1 pour une utilisation pour la lutte contre des maladies chez des animaux.

7. Comprimé selon l'une quelconque des revendications 1 à 6 pour une utilisation pour la lutte contre les helminthes chez des animaux.

8. Procédé pour la préparation des comprimés selon la revendication 1, dans lequel
(a) on mélange les principes actifs, ainsi qu'éventuellement des auxiliaires supplémentaires, on granule et éventuellement on tamise les granulés,
(b) on mélange de manière homogène l'arôme de viande éventuellement avec des auxiliaires supplémentaires et on granule à sec,
(c) on ajoute éventuellement des auxiliaires supplémentaires au mélange de (a) et (b) et on transforme le tout en un mélange homogène transformable en comprimés et
(d) on transforme ensuite le mélange en comprimés.
